# EUROPÄISCHE PATENTSCHRIFT

(11) **EP 1 395 189 B1**
(45) Veröffentlichungstag und Bekanntmachung des Hinweises auf die Patenterteilung: **02.08.2006**
(21) Anmeldenummer: 02732751.9
(22) Anmeldetag: 05.06.2002
(51) Int. Cl.: A61B 18/00

(54) **ANORDNUNG ZUM MATERIALABTRAG**
SYSTEM FOR THE REMOVAL OF MATERIAL
DISPOSITIF D'ENLEVEMENT DE MATERIAU

(30) Priorität: 15.06.2001 DE 10129650
(43) Veröffentlichungstag der Anmeldung: 10.03.2004
(73) Patentinhaber: Carl Zeiss Meditec AG, 07745 Jena (DE)
(72) Erfinder: DICK, Manfred, 07926 Gefell (DE); ELBRECHT, Jens, 07751 Drackendorf (DE); SCHRÖDER, Eckhard, 90542 Eckental (DE); SEITZ, Bernhard, 07751 Jena-Wogau (DE)
(74) Vertreter: Geyer, Fehners & Partner
(86) Internationale Anmeldenummer: PCT/EP2002/006151
(87) Internationale Veröffentlichungsnummer: WO 2002/102262

(56) Entgegenhaltungen:
- WO-A-00/33912
- WO-A-98/57588
- DE-A- 10 020 522
- US-A- 4 850 352

## Beschreibung

### Gebiet der Erfindung

Die Erfindung bezieht sich auf eine Anordnung zum Abtragen von Material mit Hilfe von Laserstrahlung.

### Stand der Technik

Verfahren und Anordnungen zum Abtragen von Material von der Oberfläche eines Gegenstandes mit Hilfe eines Laserstrahles, der auf die Oberfläche gerichtet ist, sind an sich bekannt. Darunter auch Verfahren und Anordnungen, bei denen der Laserstrahl scannend über die Oberfläche geführt und das Material dabei definiert abgetragen und dabei die Geometrie des Gegenstandes gezielt verändert wird.

Dabei kommt es insbesondere bei weichen, temperaturempfindlichen Materialien darauf an, die Laserenergie ohne wesentliche thermische Schädigung des Gebietes um den Abtragungsort einzubringen. Dies ist insbesondere dann von Bedeutung, wenn das abzutragende Material stark feuchtigkeitshaltig ist und eine Austrocknung des Materials infolge des Energieeintrages verhindert werden soll, um zu vermeiden, daß sich die Materialeigenschaften oder auch die Bedingungen für den Materialabtrag, wie etwa die Abtragungsgeschwindigkeit, während des Abtragungsprozesses in unerwünschter Weise verändern.

Das ist beispielsweise der Fall, wenn die Oberflächen krümmung einer künstlichen Kontaktlinse verstärkt oder verringert werden soll mit dem Ziel, die Sehkraft eines menschlichen Auges mit dieser Kontaktlinse zu korrigieren. Aber auch bei der Bearbeitung von totem oder lebendem biologischem Gewebe wie Knorpel, Zahnhartgewebe oder auch in der Augenchirurgie bei der Formung der Hornhaut (photorefraktive Keratektomie) kommt es nicht nur auf Sorgfalt bei der Formgebung, sondern auch darauf an, die Eigenschaften des verbleibenden Materials beizubehalten.

Von besonderer Bedeutung für einen definierten Materialabtrag bei den vorgenannten Anwendungen ist die Konstanthaltung der klimatischen Bedingungen in der Umgebung des Abtragungsortes über die Dauer des Materialabtrages hinweg, die vor allem bestimmt sind von der Temperatur und der Feuchtigkeit an der Materialoberfläche bzw. in deren unmittelbarer Nähe.

Von weiterer Bedeutung für den definierten Materialabtrag ist aber auch die Kontinuität des Energieeintrages in das Material. Da die Laserstrahlung auf dem Weg zwischen einer Abstrahlungsoptik und dem Abtragungsort die freie Atmosphäre oder gegebenenfalls auch ein Schutzgas durchquert, ist es möglich, daß die bei der Abtragung des Materials entstehenden Abprodukte, wie Rauch oder Materialpartikel, die Atmosphäre in unmittelbarer Nähe des Abtragungsortes beeinträchtigen und dabei auch, indem sie den Laserstrahl durchqueren, die Intensität der Laserstrahlung in undefinierbarer Weise schwächen.

Aus US 5,344, 418 ist eine Anordnung bekannt, bei der nahe der Austrittsöffnung für den Laserstrahl aus einem Gerät zur Materialabtragung Strömungskanäle vorgesehen sind, aus denen während des Materialabtrages ein Gas- bzw. Luftstrom auf den Abtragungsort gerichtet ist, womit erreicht wird, daß Rauch und Materialpartikel vom Abtragungsort weggeblasen werden. Nachteilig dabei ist allerdings, daß der Gas- bzw. Luftstrom die Materialoberfläche am Abtragungsort überstreicht, was zur Folge hat, daß sowohl ein auf der Oberfläche vorhandener Feuchtigkeitsfilm zerstört und damit dessen Schutzfunktion aufgehoben wird als auch insbesondere innerhalb von feuchtehaltigem, stark hygroskopischem Material eine Austrocknung in unzulässigem Maße erfolgt bzw. die Hydratationszustände im Material während des Abtrages einer unerwünschten Beeinflussung unterworfen sind.

Bei einer in US 5,181 ,916 beschriebenen Einrichtung werden die Verunreinigungen wie Rauch oder Materialpartikel nicht weggeblasen, sondern mit Hilfe eines Gasstromes abgesaugt. Zu diesem Zweck ist eine Ansaugöffnung konzentrisch um eine Mündungsöffnung eines Gerätes angeordnet, aus dem der Laserstrahl austritt und auf den Abtragungsort gerichtet ist. Doch auch hier hat das über den Behandlungsort strömende Gas zur Folge, daß sowohl die Feuchte an der Materialoberfläche abtransportiert wird als auch das Material zumindest nahe dem Abtragungsort austrocknet.

In DE 100 20 522 A1 ist eine Anordnung zur Absaugung von Abprodukten bei der Ablation von biologischem Gewebe beschrieben. Hier ist der Laserstrahl durch einen röhrenförmigen Kanal hindurch auf das Gewebe gerichtet und die Abprodukte werden in den Kanal hinein abgesaugt. Es wird innerhalb des Kanals ein gegenläufig zur Laserstrahlung gerichteter Luftstrom erzeugt, wobei die abgesaugte Luft nicht aus der Umgebung des Abtragungsortes kommt, sondern aus Zuführöffnungen strömt, die nahe der Mündungsöffnung im Kanal vorhanden sind. Das hat zur Folge, daß die Materialoberfläche nicht vom Luftstrom überstrichen und so die Austrocknung vermieden wird. Außerdem ist der Luftstrom vom Zentrum des Kanals, in dem der Laserstrahl verläuft, radial nach außen gerichtet, so daß Rauch und Materialpartikel vom Zentrum und damit vom Laserstrahl ferngehalten werden und somit verhindert wird, daß die Intensität der Laserstrahlung durch derartige Verunreinigungen in unerwünschter Weise beeinflußt wird.

WO 0033912 offenbart ein system zum Beeiflussung der Atmosphäre über einer Laserabtragestelle gemäß dem Oberbegriff des Anspruchs 1.

Allerdings gelingt es auch hiermit noch nicht, die Materialabtragung mit Laserenergie, insbesondere im Hinblick auf die Feinbearbeitung von Oberflächen, von allen Umgebungseinflüssen freizuhalten. Die Abtragungsbedingungen werden trotz der vorstehend genannten Maßnahmen immer noch durch sich während des Abtragungsvorganges verändernde Temperatur und Luftfeuchte am Abtragungsort beeinflußt. Um eine höhere Genauigkeit bei der Formgebung durch Materialabtrag erzielen zu können, besteht nach wie vor das Bedürfnis, derartige Einflüsse zu reduzieren.

### Beschreibung der Erfindung

Davon ausgehend liegt der Erfindung die Aufgabe zugrunde, unter Beibehaltung oder auch Verbesserung der bereits bekannten Maßnahmen zur Freihaltung des Laserstrahlquerschnitts von Verunreinigungen auch die klimatischen Umgebungsbedingungen am Abtragungsort über die gesamte Zeit des Abtragungsprozesses hinweg konstant zu halten.

Erfindungsgemäß wird mit den in Anspruch 1 angegebenen Mitteln die Temperatur und/oder die Feuchtigkeit am Abtragungsort und/oder in dessen unmittelbarer Nähe mittels eines während der Dauer des Abtragungsprozesses in vorgegebener Richtung über den Abtragungsort hinweg strömenden Gases im wesentlichen konstant gehalten. Das Gas hat dabei eine vorgegebene Temperatur, einen vorgegebenen Feuchtigkeitsgehalt und/oder eine vorgegebene Strömungsgeschwindigkeit.

Ausgestaltungen der Erfindung sind in den Ansprüchen 2 bis 15 angegeben.

Bei einer ersten Ausgestaltung der Erfindung wird während der gesamten Dauer des Abtragungsprozesses ein Luftstrom mit konstanter Temperatur, konstantem Feuchtigkeitsgehalt und konstanter Strömungsgeschwindigkeit über den Abtragungsort hinweg geführt.

Damit wird unter Nutzung der Luft als Transportmedium bei entsprechend vorgewählter, unter der Solltemperatur am Abtragungsort liegender Temperatur der auf den Abtragungsort gerichteten Luft überschüssige Wärmeenergie abtransportiert. Umgekehrt hat die auf den Abtragungsort gerichtete Luft eine verhältnismäßig hohe relative Feuchte, so daß die Zufuhr von Feuchtigkeit gewährleistet ist und der Austrocknung an der Materialoberfläche und innerhalb des Materials entgegengewirkt wird. Beispielsweise kann der Luftstrom mit einer Temperatur von 37° und einer relativen Luftfeuchte von 100% bei einer Strömungsgeschwindigkeit von etwa 0,Sm/s zum Abtragungsort gerichtet werden.

In Abhängigkeit von den Eigenschaften des zu bearbeitenden Materials kann es sich als günstig erweisen, wenn der Luftstrom mit einer Temperatur im Bereich von - 20°C bis 30°C, einer relativen Luftfeuchte im Bereich von 0-100% und einer Strömungsgeschwindigkeit im Bereich von 1 m/s bis 10 m/s über den Abtragungsort hinweggeführt wird. Eine häufig bevorzugte Variante besteht darin, Luft einer Temperatur von -8°C und einer relativen Feuchte von 80 % mit ca. 3m/s über den Abtragungsort hinwegzuführen.

Damit gelingt es, die klimatischen Bedingungen während der Abtragung in verhältnismäßig engen Grenzen konstant zu halten. Wird beispielsweise während des Abtragungsprozesses eine Leistung von ca. 0,5 Watt scannend kontinuierlich in das Material eingetragen, wird zwar der überwiegende Teil dieser Leistung für die Ablation verbraucht, jedoch wird ein nicht unerheblicher Leistungsanteil in thermische Energie umgewandelt, die aber weitestgehend abtransportiert wird, so daß wie bereits beschrieben das stationäre Gleichgewicht im wesentlichen erhalten bleibt.

Die Strömungsgeschwindigkeit und die Fördermenge des Luftstroms können in Abhängigkeit von der Impulsfolgefrequenz der zur Ablation genutzten Laserstrahlung so vorgegeben sein, daß das während einer Impulsfolge ablatierte Gewebe innerhalb des Zeitraumes, der bis zum Beginn der nachfolgenden Impulsfolge vergeht, mit dem Luftstrom abtransportiert werden kann. Dies ist bei einer Impulsfolgefrequenz von 1 kHz und einer am Abtragungsort bearbeiteten Flächengröße von beispielsweise 8 mm² mit einer Strömungsgeschwindigkeit von ca. 8m/s möglich, wobei die Luftmenge etwa 40 cm³/s betragen sollte. Hierbei kann ein Schlauch mit etwa 8 mm Durchmesser genutzt werden.

In einer bevorzugten Ausgestaltung ist vorgesehen, daß ein Luftstrom mit konstanter Temperatur und konstantem Feuchtigkeitsgehalt, jedoch mit während der Dauer des Abtragungsprozesses zunehmender Strömungsgeschwindigkeit über den Abtragungsort hinweg geführt wird. Auch hierbei kann der Luftstrom beispielsweise eine Temperatur von 37° und eine relative Luftfeuchte von etwa 100% haben, wobei jedoch zu Beginn der Abtragung die Strömungsgeschwindigkeit ca. 0,2m/s beträgt und über die Dauer der Abtragung hinweg bis zu 10m/s erhöht wird. Auf diese Weise gelingt es, auch bei Energieeinträgen mit höheren Leistungswerten die überschüssige thermische Energie abzuführen.

Denkbar ist es auch, den Luftstrom mit konstanter Strömungsgeschwindigkeit über den Abtragungsort hinweg zuführen, dagegen aber während des Abtragungsprozesses die Temperatur der strömenden Luft zu verringern und/oder ihre relative Feuchte zu erhöhen. Diesbezüglich kann beispielsweise vorgesehen sein, daß die Strömungsgeschwindigkeit der Luft über den gesamten Abtragungsprozeß hinweg 0,5m/s beträgt, während die Lufttemperatur innerhalb eines Bereiches von etwa 42°C zu Anfang bis zu etwa 10°C bei Ende des Abtragungsprozesses verändert wird und die Luftfeuchte von etwa 80% zu Anfang bis auf 100% bei Ende des Abtragungsprozesses verändert wird. Damit werden noch bessere Ergebnisse bei der Einstellung eines Temperatur- und Feuchtigkeitsgleichgewichtes zwischen Material und der klimatisierten Umgebung an der Materialoberfläche erzielt als bei konstanten Temperatur- und Feuchtewerten, wodurch sich weiterhin verbesserte reproduzierbare Bedingungen bei Formgebung ergeben.

In diesbezüglichen Ausgestaltungsvarianten ist es möglich, die Veränderungen von Temperatur und/oder Luftfeuchte während des Abtragungsprozesses sowohl kontinuierlich als auch anhand vorgegebener, diskontinuierlicher Zeitplan-Funktionen vorzunehmen.

Alternativ hierzu ist es auch denkbar, die Veränderung der Temperatur, der relativen Feuchte und/oder der Strömungsgeschwindigkeit der Luft in Abhängigkeit von Temperatur- und/oder Feuchtigkeitswerten vorzunehmen, die während des Abtragungsprozesses unmittelbar am oder nahe dem Abtragungsort wiederholt gemessen, ausgewertet und als Führungsgrößen für vorzunehmende Veränderungen des Luftstrom genutzt werden. So liefert beispielsweise eine kontinuierliche Messung von Temperatur und Feuchtigkeit am Abtragungsort Informationen, die dazu genutzt werden können, die Temperatur der Luft zu verringern bzw. deren Feuchte zu erhöhen oder auch die Strömungsgeschwindigkeit zu verändern, um in geeigneter Weise stetig aktiv auf die Konstanthaltung der klimatischen Bedingungen am Abtragungsort Einfluß nehmen zu können.

In Verbindung mit den vorgenannten Maßnahmen zur Konstanthaltung der Umgebungsbedingungen ist außerdem der Luftstrom stets so gerichtet, daß die während der Abtragung entstehenden Abprodukte, wie Rauch und Materialpartikel, vom Luftstrom erfaßt und mit der strömenden Luft vom Abtragungsort entfernt werden, ohne dabei den auf den Abtragungsort gerichteten Laserstrahl zu durchqueren.

Es sei ausdrücklich darauf hingewiesen, daß sich die Erfindung nicht auf die Verwendung von Luft als Transportmittel von Wärmeenergie und Feuchtigkeit beschränkt, sondern daß darüber hinaus auch jedes andere geeignete Gas, wie beispielsweise Stickstoff, verwendet werden kann.

Die erfindungsgemäße Anordnung ist bevorzugt zur Veränderung der Oberflächenkrümmung von künstlichen Kontaktlinsen geeignet mit dem Ziel, je nach Verstärkung oder Verringerung der Krümmung die Fehlsichtigkeit eines menschlichen Auges zu korrigieren. Dabei besteht ein wesentlicher Vorteil darin, daß die Bearbeitung bei Abwesenheit des späteren Kontaktlinsenträgers erfolgen kann.

Die Erfindung bezieht sich auf Anordnungen zum Abtragen von Material, die in der beschriebenen Weise die Bearbeitung sowohl von künstlichen als auch natürlichen Materialien, darunter auch biologischem Gewebe, erlauben. Bei diesen Anordnungen sind Mittel vorgesehen, mit denen während der Einwirkung der Laserenergie ein Gasstrom über den Abtragungsort hinweg geführt wird, wobei dessen Temperatur, relative Feuchte und/oder Strömungsgeschwindigkeit des über den Abtragungsort hinwegströmenden Gases vorgegeben werden können. Als Gas kommt hier bevorzugt Luft zur Anwendung, jedoch sind auch andere Gase geeignet, wie beispielsweise Stickstoff.

In einer besonders bevorzugten Ausgestaltung sind die Anordnungen ausgestattet mit Mitteln zu Vorauswahl der Temperatur, der relativen Luftfeuchte und/oder der Strömungsgeschwindigkeit aus vorgegebenen Wertebereichen. Die Auswahl kann vor Beginn des Abtragungsprozesses stattfinden, wobei Einrichtungen zum Konstanthalten der vorausgewählten Werte während des gesamten Abtragungsprozesses vorhanden sind.

Weiterhin ist vorgesehen, daß die Mittel bzw. Einrichtungen zur Vorauswahl oder Veränderung der Temperatur, der relativen Luftfeuchte und/oder der Strömungsgeschwindigkeit mit einer Ansteuerschaltung gekoppelt sind, die beispielsweise in Abhängigkeit von Werten, die nach einer Zeitplan-Funktion vorgegeben werden, Stellsignale abgeben. Diese Ansteuerschaltung kann weiterhin mit einer Lufterwärmungseinrichtung und/oder mit einer Luftbefeuchtungseinrichtung gekoppelt sein.

Die Luftbefeuchtungseinrichtung sollte vorteilhaft mit einem Vernebler, bevorzugt mit einem Ultraschallvernebler ausgestattet sein, der Feuchtigkeit mit einer konstanten Tröpfchengröße von < 4 µm abgibt. Dies gilt beispielsweise für die refraktive Laserchirurgie unter Nutzung einer Laserstrahlung der Wellenlänge von 193 nm, wobei die Vernebelungsleistung 0,5 bis 2 ml/min betragen sollte. Damit wird eine im Hinblick auf die benötigte Anfeuchtung und geringste Wasserausscheidung am Operationsort optimierte Nebeldichte erreicht.

Weiterhin sind Mittel vorgesehen, die die Strömungsrichtung des Gases bzw. der Luft derart beeinflussen, daß der Laserstrahlquerschnitt nicht durch Ablationsabprodukte durchquert und dadurch die Strahlungsintensität in undefinierbarer Weise beeinflußt wird. Diesbezüglich sind beispielhaft zwei jeweils ringförmig zentrisch um den Laserstrahl angeordnete Strömungskanäle in Richtung der Laserstrahlung aufeinander folgende vorhanden, von denen einer mit Austrittsöffnungen, der andere mit Eintrittsöffnungen für den Luftstrom ausgestattet sind. Dabei sind die Austrittsöffnungen eines ersten der beiden Strömungskanäle und die Eintrittsöffnungen des anderen Strömungskanals so positioniert, daß der Luftstrom im wesentlichen parallel zur Laserstrahlung gerichtet ist, bevorzugt mit einer Strömungsrichtung entgegengesetzt zur Richtung der Laserstrahlung.

Je nach Anwendungsfall kann es sich weiterhin als vorteilhaft erweisen, wenn die Richtung der zuströmenden Luft mit der Tangente über dem Abtragungsort einen Winkel von 0-70° einschließt und die Eintrittsöffnungen für die Absaugung des Luftstromes vom Abtragungsort so ausgebildet sind, daß die Richtung der abströmenden Luft mit der Tangente über dem Abtragungsort ebenfalls einen Winkel im Bereich zwischen 0° und 70° einschließt.

Zur Ermittlung der Feuchtigkeitswerte am Abtragungsort ist beispielsweise eine Streulichtmeßeinrichtung vorgesehen, bei der die Intensität der Reflexion eines gesonderten, auf die Materialoberfläche gerichteten Laserstrahls, dessen Wellenlänge im sichtbaren oder infraroten Spektralbereich liegt, als Maß der Feuchtigkeit an der Oberfläche genutzt wird. Die physikalischen Parameter dieser gesonderten Laserstrahlung, insbesondere Intensität und Wellenlänge, sind dabei in Abhängigkeit von den Eigenschaften des zu bearbeitenden Materials so gewählt, daß keine Veränderung der Materialeigenschaften aufgrund dieser Strahlung eintritt. Zum kontaktfreien Messen der aktuellen Temperaturen an der Materialoberfläche während des Abtragungsprozesses kann eine handelsübliche Thermokamera vorgesehen sein.

### Kurze Beschreibung der Zeichnungen

Die Erfindung soll nachfolgend anhand eines Ausführungsbeispieles näher erläutert werden. In den zugehörigen Zeichnungen zeigen
- Fig.1: eine Anordnung zum Materialabtrag mit Hilfe von Laserstrahlung, bei der ein Laserstrahl auf die Materialoberfläche gerichtet und eine Einrichtung zur Klimatisierung der Umgebung an der Oberfläche zur Zuführung eines klimatisierten Luftstromes vorgesehen ist,
- Fig.2: ein Beispiel für die Ausgestaltung einer Zu- und Abführeinrichtung für einen klimatisierten Luftstrom zur Materialoberfläche und deren Anordnung in der Nähe des Abtragungsortes,
- Fig.3: eine Möglichkeit für die Positionierung von Meßeinrichtungen zur Ermittlung von Temperatur- und Feuchtigkeitswerten aus der Nähe des Abtragungsortes.
- Fig.4: eine weitere Ausgestaltungsmöglichkeit der Einrichtung zur Zuführung und Abführung eines klimatisierten Luftstromes.

### Ausführliche Beschreibung der Zeichnungen

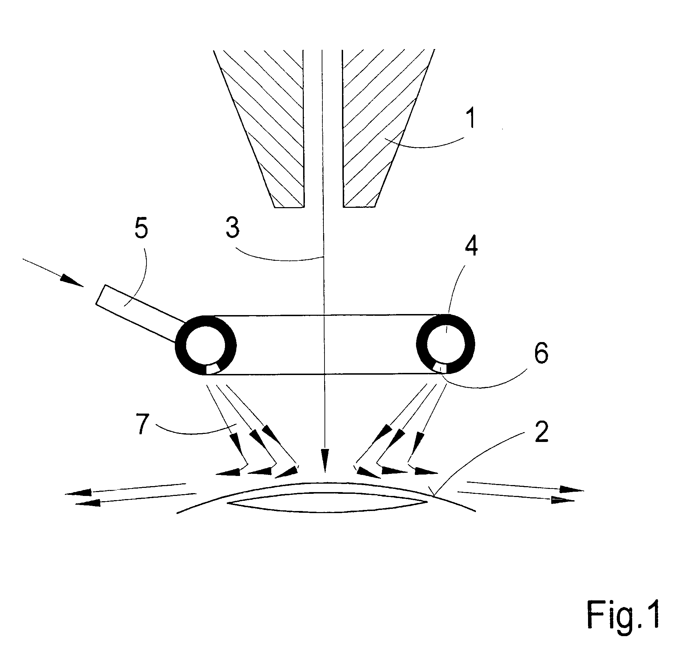

In Fig.1 ist in einer nicht unter den Schutz dieses Patentes fallenden Anordnung zum Materialabtrag ein röhrenförmiger Kanal 1 dargestellt, aus dessen Ende ein Laserstrahl 3 austritt und auf die Oberfläche eines Gegenstandes 2 gerichtet ist. Eine solche Anordnung kann beispielsweise zur Veränderung der Krümmung von Kontaktlinsen oder auch zur photorefraktiven Keratektomie genutzt werden, bei der die Krümmung der Hornhaut eines menschlichen Auges unter Einwirkung der Laserstrahlung gezielt durch Abtrag des biologischen Gewebes der Hornhaut korrigiert wird.

Es sei angenommen, daß während der Dauer des Ablationsprozesses ein Leistungseintrag in das Gewebe von ca. 0,5 Watt erfolgt. Dabei wird ein Großteil der Leistung für die Ablation verbraucht, ein nicht unerheblicher Teil jedoch wird in unerwünschte thermische, akustische und Fluoreszenzenergie umgewandelt. Dabei würden vor allem die thermischen Verlustanteile die Abtragungsbedingungen stören, da hierdurch der Tränenfilm, also die Feuchtigkeit auf der Hornhaut sowie die Hydratationseigenschaften der Hornhaut selbst beeinflußt werden.

Um eine definierte Abtragungsrate und damit die Möglichkeit einer definierten Formgebung der Hornhautoberfläche erzielen zu können, soll der unerwünschte Einfluß auf die Ablationsbedingungen verringert bzw. nach Möglichkeit ganz aufgehoben werden, indem die klimatischen Umgebungsbedingungen konstant gehalten werden.

Diesbezüglich ist ein röhrenförmiger Kanal 4 vorgesehen, der über einen Anschlußstutzen 5 und eine hieran angeschlossene (nicht dargestellte) Verbindungsleitung mit einer Luftfördereinrichtung (ebenfalls nicht dargestellt) in Verbindung steht.

Die Luftfördereinrichtung sorgt dafür, daß dem röhrenförmigen Strömungskanal 4 Luft zugeführt wird, die durch Austrittsöffnungen 6 aus dem Strömungskanal 4 austritt.

Dabei sind die Austrittsöffnungen 6 so angeordnet, daß die Strömungsrichtungen 7 der austretenden Luft, mit dem Laserstrahl 3 einem spitzen Winkel einschließend, zur Oberfläche des Gegenstandes 2 hin gerichtet sind.

Der Strömungskanal 4 ist ringförmig gekrümmt und zentrisch um den Laserstrahl 3 angeordnet, während die Austrittsöffnungen 6 radialsymmetrisch um den Laserstrahl verteilt positioniert sind, so daß die Strömungsrichtungen 7 in ihrer Gesamtheit etwa einen Kreiskegelmantel bilden, in dessen Zentrum der Laserstrahl 3 verläuft.

Der Abstand des Strömungskanals 4 zum Gegenstand 2 ist so bemessen, daß die Spitze dieses Kreiskegelmantels etwa mit dem Auftreffort des Laserstrahles 3 auf dem Gegenstand 2 zusammenfällt.

Damit wird erreicht, daß Strömungsrichtungen 7 etwa am Auftreffort des Laserstrahles 3 auf der Gegenstand 2 aufeinandertreffen und sich dabei gegenseitig so beeinflussen, daß sich die Strömungsrichtungen 7 umkehren und die strömende Luft radial nach außen abgeführt wird. Das hat zur Folge, daß Ablationsabprodukte wie Rauch und ablatierte Gewebepartikel vom Luftstrom erfaßt und mit der Luft in radialer Richtung abtransportiert werden.

Damit wird weitestgehend erreicht, daß die Ablationsprodukte den Laserstrahl 3 nicht durchqueren und so auch die Laserstrahlungsintensität nicht beeinträchtigen können.

Außerdem ist vorgesehen, daß die Luftfördereinrichtung mit einer Klimatisierungseinrichtung für die zum Strömungskanal 4 geleitete Luft gekoppelt ist. Die Klimatisierungseinrichtung ist so ausgebildet, daß Temperatur und relative Feuchte der Luft beeinflußt werden können. Außerdem sind Mittel vorhanden, mit denen die Temperaturwerte, Werte für die relative Luftfeuchte sowie auch Werte für die je Zeiteinheit geförderte Luftmenge vor Beginn des Ablationsprozesses voreingestellt werden können. Diesbezüglich ist sowohl die Luftfördereinrichtung als auch die Klimatisierungseinrichtung mit Mitteln zur Befehlseingabe, beispielsweise Tastern, Schaltern oder Drehgebern ausgestattet, die Teil einer Ansteuerung sind. Derartige Einrichtungen zur Luftförderung und zur Klimatisierung der Luft sowie entsprechende Eingabemittel sind aus dem Stand der Technik bekannt und müssen deshalb hier nicht näher erläutert werden.

Ist beispielsweise als Quelle für die Laserstrahlung ein Excimer-Laser, vorzugsweise des Typs MEL 70 G-Scan, vorgesehen, der die sehr sensible Laserstrahlung mit einer Wellenlänge von 193 nm abgibt, so kann durch Vorauswahl einer Temperatur von 37°C, einer relativen Luftfeuchte von etwa 10096 und einer Strömungsgeschwindigkeit von etwa 0,5m/s dafür gesorgt werden, daß die klimatischen Umgebungsbedingungen um den Abtragungsort während des Ablationsprozesses in verhältnismäßig engen Grenzen konstant bleiben. Damit wird auch eine verhältnismäßig konstante Abtragungsrate gewährleistet, womit die erforderliche Präzision bei der Formgebung weitestgehend erreicht ist.

Zusätzlich können sowohl die Luftfördereinrichtungen als auch die Klimatisierungseinrichtungen noch mit Mitteln zur Konstanthaltung der vorgewählten Werte ausgestattet sein. Derartige Einrichtungen, die sowohl die Temperatur, die Luftfeuchte als auch die Strömungsgeschwindigkeit von Luft konstant halten, sind ebenfalls aus dem Stand der Technik bekannt und werden deshalb hier nicht näher erläutert.

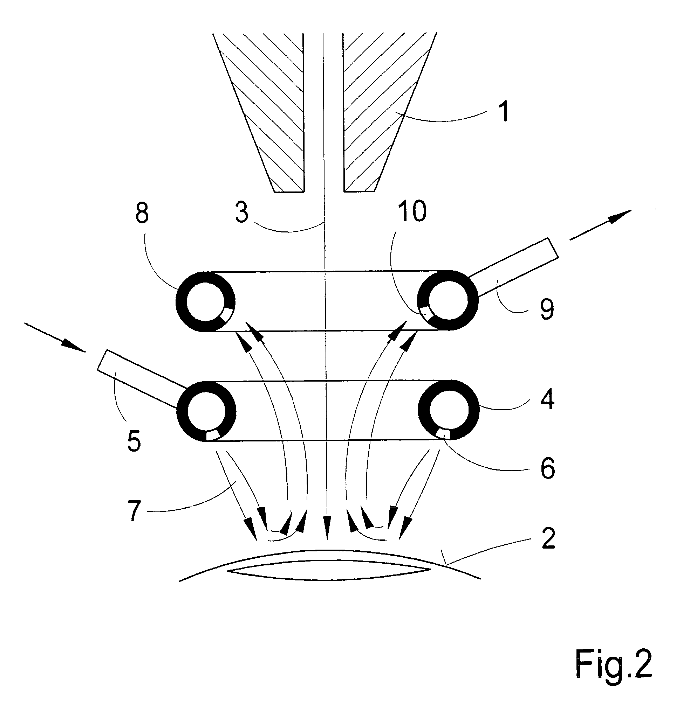

In einem in Fig.2 dargestellten Ausgestaltungsbeispiel der erfindungsgemäßen Anordnung ist neben dem röhrenförmigen Strömungskanal 4 ein weiterer röhrenförmiger Strömungskanal 8 vorgesehen, der ebenso wie der Strömungskanal 4 ringförmig um den Laserstrahl 3 angeordnet ist, der sich jedoch in einer größeren Entfernung als der Strömungskanal 4 von dem Gegenstand 2 befindet und im Gegensatz zum Strömungskanal 4 nicht mit einer Luftfördereinrichtung zur Zuführung von Luft, sondern mit einer Absaugeinrichtung (zeichnerisch nicht dargestellt) verbunden ist, die über eine Schlauchleitung (ebenfalls zeichnerisch nicht dargestellt) und einen Anschlußstutzen 9 mit dem Strömungskanal 8 in Verbindung steht.

Der Strömungskanal 8 verfügt über Eintrittsöffnungen 10, die im wesentlichen in gleicher Ordnung wie die Austrittsöffnungen 6 am Strömungskanal 4 positioniert sind.

Beim Betreiben dieser Anordnung entsteht rings um den Laserstrahl 3 eine Luftströmung, die zunächst aus den Austrittsöffnungen 6 des Strömungskanals 4 zum Gegenstand 2 und danach vom Gegenstand 2 zu den Einströmöffnungen 10 des Strömungskanals 8 hingerichtet ist.

lm Gegensatz zur Ausgestaltungsvariante nach Fig.1 wird mit dieser Anordnung dafür gesorgt, daß die Ablationsabprodukte nicht radial vom Laserstrahl 3 nach außen abtransportiert werden, sondern (etwa in entgegengesetzter Richtung zum Laserstrahl 3) durch die Eintrittsöffnungen 10 hindurch in den Strömungskanal 8 hinein und von dort zur Absaugeinrichtung abtransportiert werden. Dies hat zur Folge, daß die Ablationsabprodukte (Rauch, Gewebepartikel) den Laserstrahl 3 nicht durchqueren können und außerdem auch nicht die Umgebung des Ablationsortes verunreinigen bzw. zu Geruchsbelästigungen für die bearbeitende Person führen.

Hierbei ist vorgesehen, daß die Luft mit vorgegebener, konstant gehaltener Temperatur, relativen Feuchte und Strömungsgeschwindigkeit aus den Ausströmöffnungen 6 austritt und auf diese Weise für definierte klimatische Bedingungen an dem Gegenstand 2 sorgt.

Dagegen kann in einer weiteren Ausgestaltung der Anordnung vorgesehen sein, daß die Temperatur- und Feuchtigkeitswerte der Luft sowie deren Strömungsgeschwindigkeit während des Abtragungsprozesses nicht konstant gehalten werden, sondern daß Meßwertaufnehmer zur Erfassung von Temperatur- und Feuchtigkeitswerten aus unmittelbarer Nähe des Abtragungsortes vorhanden und über eine Ansteuerung mit der Luftfördereinrichtung und der Klimatisierungseinrichtung in Verbindung stehen.

Damit ist es möglich, auf aktuelle Veränderungen der klimatischen Umgebungsbedingungen sehr schnell zu reagieren, indem auf Grundlage der ermittelten Werte die Erhöhung oder Verringerung der Temperatur der strömenden Luft oder auch deren relativer Feuchte veranlaßt wird, um damit in noch engeren Grenzen der Auswirkung der thermischen Verlustleistung entgegenwirken zu können.

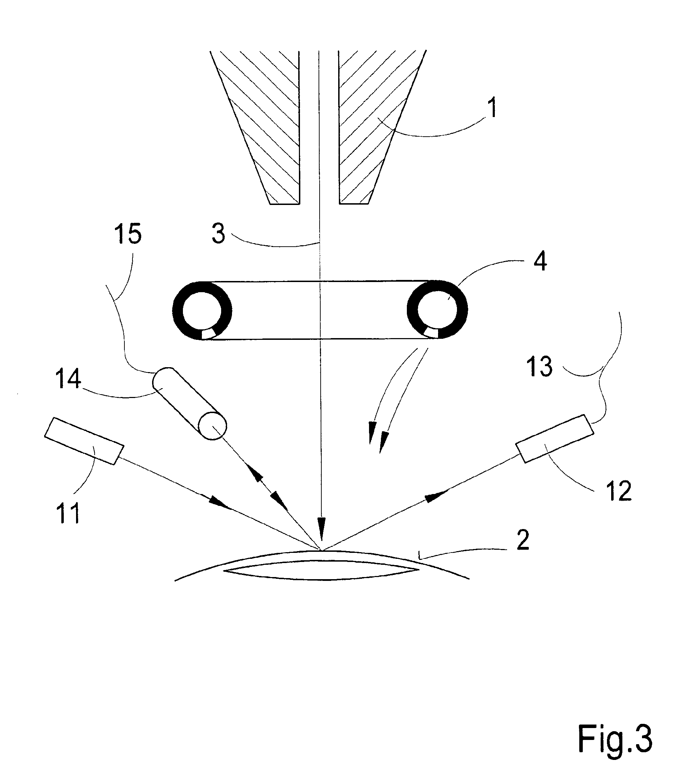

Beispiele für die Anordnung solcher Meßwertgeber sind in Fig.3 anhand einer nicht unter den Schutz dieses Patentes fallenden Anordnung zum Materialabtrag dargestellt. Hier ist beispielsweise zum Messen der Feuchtigkeitswerte am Abtragungsort eine Streulichtmeßeinrichtung vorgesehen, bestehend aus einer Laserdiode 1 1 , die Licht im sichtbaren oder infraroten Spektralbereich auf den Gegenstand 2 richtet, und aus einem Fotodetektor 12, der die Reflexion des von der Laserdiode 1 1 ausgesendeten Laserstrahlung empfängt und dessen Ausgangssignale ein Maß für die Feuchtigkeit an der Hornhautoberfläche sind.

Über einen Signalweg 13 steht die Fotodiode 12 über eine Auswerte- und Ansteuerschaltung (nicht dargestellt) mit der Klimatisierungseinrichtung in Verbindung. Die reflektierte gestreute Intensität der von der Fotodiode 11 ausgehenden Laserstrahlung wird wesentlich davon bestimmt, ob sich noch ein Feuchtigkeitsfilm auf der Hornhautoberfläche befindet bzw. wie weit bereits eine Austrocknung erfolgt ist.

Zur Gewinnung von Temperaturwerten aus der unmittelbaren Nähe des Abtragungsortes kann eine handelsübliche Temperaturfernmeßeinrichtung, etwa eine Thermokamera, verwendet und mit ihrer Meßrichtung so ausgerichtet werden, daß damit die Temperaturwerte vom Abtragungsort erfaßt und über einen Signalweg 14 an die Auswerte- und Ansteuerschaltung weitergegeben werden, die mit der Klimatisierungseinrichtung in Verbindung steht.

Mit der vorliegenden Erfindung wird erreicht, daß mit einer kompakten Anordnung in der unmittelbaren Nähe des Behandlungsortes, beispielsweise des zu behandelnden Auges bei der photorefraktiven Keratektomie, neben der Absaugung der Ablationsabprodukte eine definierte Temperatur und Luftfeuchte eingestellt werden kann und damit bewirkt wird, daß die Abtragungseigenschaften des Hornhautgewebes konstant gehalten werden. Wie ausführlich dargelegt, erfolgt die Einstellung des stationären Gleichgewichtes von Luftfeuchte und Temperatur während der Laserbehandlung durch gezielte Zu- und Abfuhr temperierter, befeuchteter Luft bei definierter Strömungsgeschwindigkeit in der unmittelbaren Nähe des Behandlungsortes.

Mit einer bevorzugten Ausgestaltung wird mit gesättigter Luft, die etwa auf Körpertemperatur erwärmt ist, und einer verhältnismäßig geringen Strömungsgeschwindigkeit von etwa 0,5 m/s über den gesamten Verlauf des Ablationsprozesses hinweg gearbeitet. Ein zu Beginn des Abtragungsprozesses noch auf den Gegenstand 2 vorhandener Feuchtigkeitsfilm bedingt eine zunächst geringe Ablationsrate. Da aber während des Ablationsprozesses thermische Energie frei wird, führt diese dazu, daß der Feuchtigkeitsfilm zunehmend austrocknet und sich die Ablationsrate dadurch erhöht. Dem wird in der beschriebenen Art und Weise entgegengewirkt.

Bei der Arbeit mit einem Excimer-Laser als Strahlungsquelle für die Wellenlänge von 193 nm ist zu beachten, daß die Absorption für diese Wellenlänge in Wasser deutlich höher ist als in Luft bzw. einem anderen Spülgas, wie beispielsweise etwa Stickstoff. Daraus wird deutlich, daß es erforderlich ist, der Austrocknung entgegenzuwirken, um zu konstanten Abtragungsbedingungen zu kommen. Insofern gelingt es, stets ein Temperatur- und Feuchtigkeitsgleichgewicht zwischen dem Gegenstand (Kontaktlinse oder Hornhaut) und klimatisierter Umgebung an dessen Oberfläche einzustellen. Störgrößen wie ein anfänglich stärker vorhandener Feuchtigkeitsfilm sowie die mit dem Energieeintrag zunehmende Austrocknung werden mit den erfindungsgemäß vorgeschlagenen Mitteln ausgeglichen.

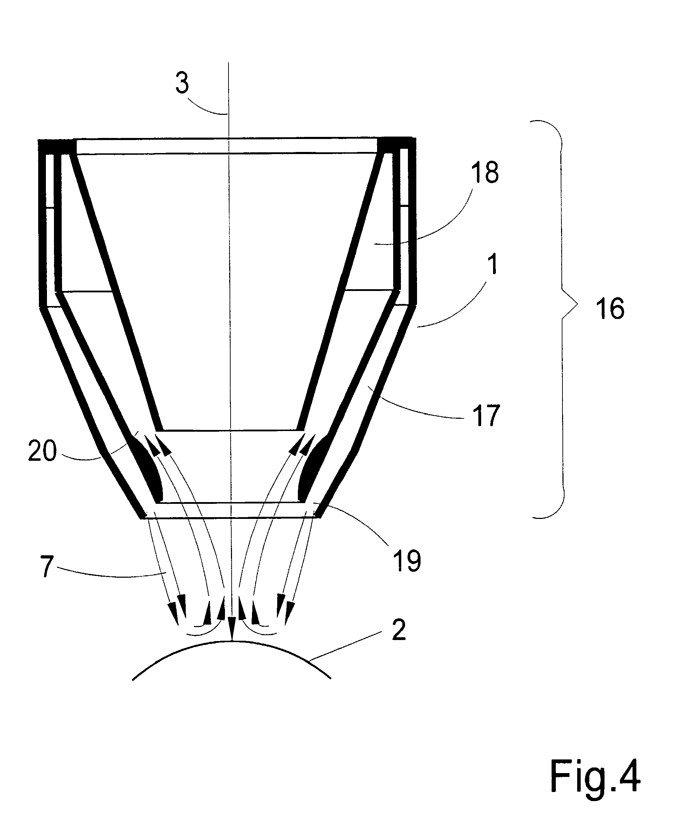

Fig.4 zeigt eine weitere Ausgestaltungsmöglichkeit hinsichtlich der Zuführung und Abführung eines klimatisierten Luftstromes 7 zur bzw, von der Oberfläche des Gegenstandes 2. Wie aus Fig.4 hervorgeht, weist das dem Gegenstand 2 zugewandte Ende des röhrenförmigen Kanals 1 einen konisch verlaufenden Abschnitt 16 mit zwei den Laserstrahl 3 konzentrisch umschließenden Kammern 17 und 18 auf. Die Kammer 17, die in eine ringförmige Ausströmöffnung 19 mündet, steht mit einer Luftklimatisierungs- und -fördereinrichtung (zeichnerisch nicht dargestellt) in Verbindung, die der Erzeugung eines Überdrucks aus klimatisierter Luft in der Kammer 17 dient. Die ringförmige Ausströmöffnung 19 ist so gestaltet, daß der aufgrund des Überdrucks austretende klimatisierte Luftstrom 7 auf die Oberfläche des Gegenstandes 2 gerichtet ist, wo er reflektiert wird.

Die Kammer 18 ist mit einer Absaugeinrichtung (zeichnerisch nicht dargestellt) verbunden, die einen Unterdruck erzeugt, und sie weist eine ringförmige Einströmöffnung 20 auf, durch welche der von der Oberfläche des Gegenstandes 2 reflektierte Luftstrom 7 in die Kammer 18 hineingesaugt und zur Absaugeinrichtung abtransportiert wird.

Zur Verbindung der Kammer 17 mit der Luftklimatisierungs- und -fördereinrichtung und zur Verbindung der Kammer 18 mit der Absaugeinrichtung können jeweils Schlauchleitungen vorgesehen sein, die über Anschlußstutzen anzuschließen sind. Als Luftklimatisierungs- und -fördereinrichtung und als Absaugeinrichtung sind handelsübliche Baugruppen nutzbar, so daß eine nähere Erläuterung hier entfallen kann.

Mit der Ausgestaltung nach Fig.4 wird ebenfalls wie schon mit der Anordnung nach Fig.2 erreicht, daß die Ablationsprodukte abgesaugt werden, ohne daß diese den Laserstrahl 3 durchqueren und so die Intensität der Laserstrahlung beeinträchtigen können. Die Oberfläche des Gegenstandes 2 kann aufgrund des klimatisierten Luftstromes 7 nicht austrocknen, wodurch gleichbleibende Abtragungsbedingungen gewährleistet sind.

## Patentansprüche

1. Anordnung zum Abtragen von Material mit Hilfe von Laserstrahlung von der Oberfläche eines Gegenstandes (2), wobei
- mittels einer Luftfördereinrichtung und einer Absaugeinrichtung während der Dauer des Abtragungsprozesses ein Luftstrom über den Abtragungsort hinweg geführt wird, **dadurch gekennzeichnet, dass**
- die Luftfördereinrichtung mit Austrittsöffnungen (6) und die Absaugeinrichtung mit Eintrittsöffnungen (10) für den Luftstrom ausgestattet sind und
- die Austrittsöffnungen (6) und Eintrittsöffnungen (10) an jeweils ringförmig zentrisch um den Laserstrahl (3) angeordneten Strömungskanälen (4,8) ausgebildet und 50 zur Oberfläche des Gegenstandes (2) positioniert sind, daß die Richtung der auf die Oberfläche zuströmenden Luft mit der über dem Abtragungsort an die Oberfläche gelegten Tangente einen Winkel von 0° bis 70° einschließt, während die Laserstrahlung mit der Tangente einen rechten Winkel einschließt, so daß die Luft mit der Oberfläche in Kontakt tritt, und auch die Richtung der von der Oberfläche abströmenden Luft mit der Tangente über dem Abtragungsort einen Winkel von 0° bis 70° einschließt, und wobei
- Mittel zur Beeinflussung der Temperatur, der relativen Luftfeuchte und/oder der Strömungsgeschwindigkeit der über den Abtragungsort hinwegströmenden und dabei mit der Oberfläche in Kontakt tretenden Luft vorgesehen sind.

2. Anordnung nach Anspruch 1, **dadurch gekennzeichnet, daß** Mittel zur Vorauswahl der Temperatur, der relativen Luftfeuchte und/oder der Strömungsgeschwindigkeit aus vorgegebenen Wertebereichen vor Beginn des Abtragungsprozesses vorgesehen sind und Einrichtungen zum Konstanthalten der vorausgewählten Werte während des Abtragungsprozesses vorhanden sind.

3. Anordnung nach Anspruch 1 oder 2, **dadurch gekennzeichnet, daß** die Einrichtungen zum Konstanthalten
- der Temperatur bei etwa minus 8°C,
- der relativen Luftfeuchte bei etwa 80%
- und der Strömungsgeschwindigkeit bei etwa 3 m/s ausgebildet sind.

4. Anordnung nach Anspruch 1, **dadurch gekennzeichnet, daß** Einrichtungen zum Verändern der Temperatur, der relativen Luftfeuchte und/oder der Strömungsgeschwindigkeit der über den Abtragungsort hinweg strömenden Luft während des Abtragungsprozesses innerhalb vorgegebener Wertebereiche vorgesehen sind.

5. Anordnung nach Anspruch 4, **dadurch gekennzeichnet, daß** die Einrichtungen mit Ansteuerschaltungen gekoppelt sind, durch die Veränderungen von Temperatur, relativer Luftfeuchte und/oder Strömungsgeschwindigkeit während des Abtragungsprozesses anhand vorgegebener Zeitplan-Funktionen veranlaßt werden.

6. Anordnung nach Anspruch 4, **dadurch gekennzeichnet, daß** Meßwertaufnehmer zur Messung von Temperatur- und/oder Feuchtigkeitswerten aus der unmittelbaren Umgebung des Abtragungsortes vorgesehen und über Auswerteeinrichtungen mit Ansteuerschaltungen verknüpft sind, durch die Veränderungen von Temperatur, relativer Luftfeuchte und/oder Strömungsgeschwindigkeit in Abhängigkeit von den gemessenen Werten veranlaßt werden.

7. Anordnung nach einem der Ansprüche 4 bis 6, **dadurch gekennzeichnet, daß** Veränderungen im Verlaufe des Abtragungsprozesses wie folgt vorgesehen sind
- der Temperatur im Wertebereich von minus 20°C bis plus 30°C,
- der relativen Luftfeuchte im Wertebereich von 0 bis 100%, und/oder
- der Strömungsgeschwindigkeit im Wertebereich von 1 m/s bis 10 m/s.

8. Anordnung nach einem der Ansprüche 1 bis 7, **dadurch gekennzeichnet, daß** eine mit einer Ansteuerschaltung gekoppelte Lufterwärmungseinrichtung vorhanden ist.

9. Anordnung nach einem der Ansprüche 1 bis 8, **dadurch gekennzeichnet, daß** eine mit einer Ansteuerschaltung gekoppelte Luftbefeuchtungseinrichtung vorhanden ist.

10. Anordnung nach Anspruch 9, **dadurch gekennzeichnet, daß** die Luftbefeuchtungseinrichtung mit einem Vernebler, bevorzugt einem Ultraschallvernebler ausgestattet ist, der 0,5 ml bis 2 ml Wasser pro Minute vernebelt bei einer Tröpfengröße von <4 µm.

11. Anordnung nach einem der Ansprüche 1 bis 10, **dadurch gekennzeichnet, daß** eine mit einer Ansteuerschaltung gekoppelte Luftfördereinrichtung vorhanden ist.

12. Anordnung nach Anspruch 1, **dadurch gekennzeichnet, daß** die Austrittsöffnungen (6) so positioniert sind, daß der Luftstrom entgegengesetzt zur Laserstrahlung gerichtet ist.

13. Anordnung nach Anspruch 6, **dadurch gekennzeichnet, daß** zum Messen von Feuchtigkeitswerten eine Streulichtmeßeinrichtung vorgesehen ist, bei der die Intensität der Reflexion eines Laserstrahls mit Wellenlängen im sichtbaren oder infraroten Spektralbereich an der Materialoberfläche als Maß der Feuchtigkeit an der Materialoberfläche genutzt wird.

14. Anordnung nach Anspruch 6, **dadurch gekennzeichnet, daß** zum kontaktfreien Messen der aktuellen Temperaturen an der Materialoberfläche eine Thermokamera (14) vorgesehen ist.

## Claims

1. Arrangement for the removal of material with the aid of laser radiation from the surface of an object (2) wherein
- an air flow is guided over the removal site by means of an air conveying device and a suction device for the duration of the removal process,
**characterised in that**
- the air conveying device is equipped with outlet opening s(6) and the suction device is equipped with inlet openings (10) for the air flow, and
- the outlet openings (6) and the inlet openings (10) are constructed on flow channels (4, 8) disposed in each case in the form of a ring centrally abut the laser beam (3) and are positioned relative to the surface of the object (2) so that the direction of the air flowing in onto the surface encloses an angle of 0° to 70° with the tangent applied to the surface via the removal site, whilst the laser radiation encloses a right angle with the tangent, so that the air comes into contact with the surface, and also the direction of the air flowing off from the surface encloses an angle of 0° to 70° with the tangent to the removal site, and wherein
- means for influencing the temperature, the relative humidity and/or the flow velocity of the air flowing over the removal site and thus coming into contact with the surface.

2. Arrangement as claimed in Claim 1, **characterised in that** means are provided for preselection of the temperature, the relative humidity and/or the flow velocity from predetermined value ranges before the beginiling of the removal process and devices are provided for keeping the preselected values constant during the removal process.

3. Arrangement as claimed in Claim 1 or 2, **characterised in that** the devices are designed for keeping constant
- the temperature at approximately minus 8 °,
- the relative humidity at approximately 80%,
- and the flow velocity at approximately 3 m/s.

4. Arrangement as claimed in Claim 1, **characterised in that** devices are provided for changing the temperature, the relative humidity and/or the flow velocity of the air flowing across the removal site during the removal process within predetermined value ranges.

5. Arrangement as claimed in Claim 4, **characterised in that** the devices are coupled to control circuits by which the changes of temperature, relative humidity and/or flow velocity are effected with the aid of predetermined time schedule functions.

6. Arrangement as claimed in Claim 4, **characterised in that** measured value sensors are provided for the measurement of temperature and/or humidity values from the immediate surroundings of the removal site and are linked by way of evaluation devices to control circuits by which the changes of temperature, relative humidity and/or flow velocity are effected as a function of the measured values.

7. Arrangement as claimed in any one of Claims 4 to 6, **characterised in that** changes are made in the course of the removal process as follows:
- to the temperature in the value range from minus 20 °C to plus 30 °C,
- to the relative humidity in the value range from 0 to 100%, and/or
- to the flow velocity in the value range from 1 m/s to 10 m/s.

8. Arrangement as claimed in any one of Claims 1 to 7, **characterised in that** an air heater is coupled to a control circuit is provided.

9. Arrangement as claimed in any one of Claims 1 to 8, **characterised in that** an air humidifier is coupled to a control circuit is provided.

10. Arrangement as claimed in Claim 9, **characterised in that** the air humidifier is equipped with an atomiser, preferably an ultrasonic atomiser, which atomises 0.5 ml to 2 ml water per minute at a drop size of < 4 µm.

11. Arrangement as claimed in any one of Claims 1 to 10, **characterised in that** an air conveying device is coupled to a control circuit.

12. Arrangement as claimed in Claim 1, **characterised in that** the outlet openings (6) are positioned so that the air flow is in the opposite direction to the laser radiation.

13. Arrangement as claimed in Claim 6, **characterised in that** for the measurement of moisture values a scattered light measuring device is provided in which the intensity of the reflection of a laser beam with wavelengths in the visible or infrared spectral range on the material surface is used as a measurement of the moisture on the material surface.

14. Arrangement as claimed in Claim 6, **characterised in that** a thermal camera (14) is provided for contact-free measurement of the current temperatures on the material surface.

## Revendications

1. Dispositif pour l'enlèvement de matière à l'aide de rayonnement laser depuis la surface d'un objet (2), dans lequel
au moyen d'un dispositif de transport pneumatique et d'un dispositif d'aspiration, un courant d'air est guidé par-dessus et au-delà du lieu d'enlèvement pendant la durée du processus d'enlèvement, **caractérisé en ce que**
le dispositif de transport pneumatique est doté d'ouvertures de sortie (6) et le dispositif d'aspiration d'ouvertures d'entrée (10) pour le courant d'air et
les ouvertures de sortie (6) et les ouvertures d'entrée (10) sont formées sur des canaux d'écoulement (4, 8) disposés respectivement de façon annulaire dans l'axe autour du faisceau laser (3) et sont positionnées par rapport à la surface de l'objet (2) de telle sorte que la direction de l'air affluant sur la surface limite avec la tangente disposée sur la surface au-dessus du lieu d'enlèvement un angle de 0° à 70°, tandis que le rayonnement laser limite avec la tangente un angle droit, de telle sorte que l'air arrive en contact avec la surface, et aussi la direction de l'air s'écoulant depuis la surface limite avec la tangente au-dessus du lieu d'enlèvement un angle de 0° à 70°, et dans lequel
des moyens d'influencement de la température, de l'humidité relative et/ou de la vitesse d'écoulement de l'air s'écoulant par-dessus et au-delà du lieu d'enlèvement et arrivant ce faisant en contact avec la surface sont prévus.

2. Dispositif selon la revendication 1, **caractérisé en ce que** des moyens sont prévus pour la présélection de la température, de l'humidité relative et/ou de la vitesse d'écoulement de l'air avant le début du processus d'enlèvement à partir de plages prédéfinies de valeurs et **en ce que** des dispositifs existent pour le maintien des valeurs présélectionnées constantes pendant le processus d'enlèvement.

3. Dispositif selon la revendication 1 ou 2, **caractérisé en ce que** les dispositifs sont formés pour le maintien constants
de la température à environ moins 8 °C
de l'humidité relative à environ 80 %
et de la vitesse d'écoulement à environ 3 m/s

4. Dispositif selon la revendication 1, **caractérisé en ce que** des dispositifs sont prévus pour la modification de la température, de l'humidité relative et/ou de la vitesse d'écoulement de l'air s'écoulant par-dessus et au-delà du lieu d'enlèvement pendant le processus d'enlèvement à l'intérieur de plages de valeurs prédéfinies.

5. Dispositif selon la revendication 4, **caractérisé en ce que** les dispositifs sont couplés à des circuits d'excitation par lesquels des modifications de la température, de l'humidité relative et/ou de la vitesse d'écoulement pendant le processus d'enlèvement sont commandées au vu de fonctions de chronologie prédéfinies.

6. Dispositif selon la revendication 4, **caractérisé en ce que** des capteurs de valeurs de mesure pour la mesure des valeurs de température et/ou d'humidité en provenance de l'environnement immédiat du lieu d'enlèvement sont prévus et sont enchaînés par des dispositifs de dépouillement à des circuits d'excitation par lesquels des modifications de la température, de l'humidité relative et/ou de la vitesse d'écoulement sont provoquées en fonction des valeurs mesurées.

7. Dispositif selon l'une des revendications 4 à 6, **caractérisé en ce que** des modifications sont prévues au cours du déroulement du processus d'enlèvement comme suit :
de la température dans la plage de valeurs de moins 20 °C à plus 30 °C, de l'humidité relative dans la plage de valeurs de 0 à 100 % et/ ou
de la vitesse d'écoulement dans la plage de valeurs de 1 m/s à 10 m/s

8. Dispositif selon l'une des revendications 1 à 7, **caractérisé en ce qu'**il existe un dispositif de réchauffage de l'air couplé à un circuit d'excitation.

9. Dispositif selon l'une des revendications 1 à 8, **caractérisé en ce qu'**il existe un dispositif d'humidification de l'air couplé à un circuit d'excitation.

10. Dispositif selon la revendication 9, **caractérisé en ce que** le dispositif d'humidification de l'air est doté d'un nébuliseur, de préférence d'un nébuliseur à ultrasons, qui nébulise 0,5 ml à 2 ml d'eau par minute avec une taille de gouttelettes inférieure à 4 µm.

11. Dispositif selon l'une des revendications 1 à 10, **caractérisé en ce qu'**il existe un dispositif de transport de l'air couplé à un circuit d'excitation.

12. Dispositif selon la revendication 1, **caractérisé en ce que** les ouvertures de sortie (6) sont positionnées de telle sorte que le courant d'air est dirigé en sens opposé du rayonnement laser.

13. Dispositif selon la revendication 6, **caractérisé en ce qu'**un dispositif de mesure de la lumière diffusée est prévu pour la mesure des valeurs d'humidité, dans lequel l'intensité de la réflexion à la surface du matériau d'un faisceau laser avec des longueurs d'onde dans la zone visible ou infrarouge du spectre est utilisée comme mesure de l'humidité à la surface du matériau.

14. Dispositif selon la revendication 6, **caractérisé en ce qu'**une thermocaméra (14) est prévue pour la mesure sans contact des températures momentanées à la surface du matériau.
